(19) Europäisches Patentamt
European Patent Office
Office européen des brevets

(11) **EP 4 559 958 A1**

(12) **EUROPEAN PATENT APPLICATION**
published in accordance with Art. 153(4) EPC

(43) Date of publication:
**28.05.2025 Bulletin 2025/22**

(21) Application number: **24852986.9**

(22) Date of filing: **18.07.2024**

(51) International Patent Classification (IPC):
*C08J 11/24* (2006.01)    *C07C 68/06* (2020.01)
*C07C 67/54* (2006.01)    *C07C 7/12* (2006.01)
*C07C 7/04* (2006.01)

(52) Cooperative Patent Classification (CPC):
**C07C 7/04; C07C 7/12; C07C 67/54; C07C 68/06;
C08J 11/24**

(86) International application number:
**PCT/KR2024/010372**

(87) International publication number:
**WO 2025/042049 (27.02.2025 Gazette 2025/09)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.08.2023 KR 20230109847**

(71) Applicant: **LG Chem, Ltd.
Yeongdeungpo-gu
Seoul 07336 (KR)**

(72) Inventors:
• **LEE, Jeongbin
  Daejeon 34122 (KR)**

• **HWANG, Jinyoung
  Daejeon 34122 (KR)**
• **KIM, Jeongnam
  Daejeon 34122 (KR)**
• **HONG, Mooho
  Daejeon 34122 (KR)**
• **OH, Sung Joon
  Daejeon 34122 (KR)**

(74) Representative: **Goddar, Heinz J.
Boehmert & Boehmert
Anwaltspartnerschaft mbB
Pettenkoferstrasse 22
80336 München (DE)**

(54) **METHOD FOR PREPARING MONOMER COMPOSITION FOR SYNTHESIZING RECYCLED PLASTIC, APPARATUS FOR PREPARING MONOMER COMPOSITION FOR SYNTHESIZING RECYCLED PLASTIC, AND MONOMER COMPOSITION FOR SYNTHESIZING RECYCLED PLASTIC, RECYCLED PLASTIC, AND MOLDED ARTICLE USING SAME**

(57)    The present disclosure relates to a preparation method of a monomer composition for synthesizing recycled plastic, the method comprising the steps of: subjecting a polycarbonate-based resin to a depolymerization reaction in the presence of an alcohol; monitoring a Raman spectrum of an aromatic diol compound obtained from the depolymerization reaction or alcohol in a reactor through which depolymerization reaction proceeds; and recovering the aromatic diol compound, and to a preparation device of a monomer composition for synthesizing recycled plastic, and a monomer composition for synthesizing recycled plastic, a recycled plastic and a molded product using the same.

【FIG. 1】

**Description**

**[TECHNICAL FIELD]**

CROSS-REFERENCE TO RELATED APPLICATION(S)

This application claims the benefit of Korean Patent Application No. 10-2023-0109847 filed on August 22, 2023 with the Korean Intellectual Property Office, the disclosure of which is incorporated herein by reference in its entirety.

The present disclosure relates to a preparation method of a monomer composition for synthesizing recycled plastic, a preparation device of a monomer composition for synthesizing recycled plastic, a monomer composition for synthesizing recycled plastic, a recycled plastic and a molded product using the same, wherein the process of recycling aromatic diol compounds through a chemical decomposition of a polycarbonate-based resin is monitored in real time to optimize reaction conditions and improve efficiency.

**[BACKGROUND]**

Polycarbonate is a thermoplastic polymer and is a plastic having excellent characteristics such as excellent transparency, superior ductility, and relatively low production costs.

Although polycarbonate is widely used for various purposes, environmental and health concerns during waste treatment have been continuously raised.

Currently, a physical recycling method is carried out, but in this case, a problem accompanying the deterioration of the quality occurs, and thus, research on the chemical recycling of polycarbonate is underway.

Chemical decomposition of polycarbonate refers to obtaining an aromatic diol compound as a monomer (e.g., bisphenol A (BPA)) through decomposition of polycarbonate, and then utilizing it again in polymerization to obtain a high-purity polycarbonate.

For such a chemical decomposition, thermal decomposition, hydrolysis, and alcohol decomposition are typically known. Among these, the most common method is alcohol decomposition using a base catalyst, but in the case of methanol decomposition, there is a problem that methanol is used which is harmful to the human body, and in the case of ethanol, there is a problem that high temperature and high pressure conditions are required and the yield is not high.

In addition, although an alcohol decomposition method using an organic catalyst is known, it is disadvantageous in terms of economics.

On the other hand, as the reaction scale in the reaction for decomposing polycarbonate increases to tens of liters, there is an emerging need to explore factors that indicate differences from the tendency to depolymerize at the laboratory level.

Conventionally, the conversion rate of the final product was analyzed through HPLC or NMR analysis, or samples were collected and analyzed during the reaction.

However, as the reaction scale increased, there was a difference in the conversion rate of polycarbonate from the reaction that proceeded at the laboratory level, and there was also a difference in the time point and extent to which side-reactants are produced.

**[DETAILED DESCRIPTION OF THE INVENTION]**

**[Technical Problem]**

It is an object of the present disclosure to provide a preparation method of a monomer composition for synthesizing recycled plastic, or a preparation device of a monomer composition for synthesizing recycled plastic, wherein the process of recycling aromatic diol compounds through a chemical decomposition of a polycarbonate-based resin is monitored in real time to optimize reaction conditions and improve efficiency.

It is another object of the present disclosure to provide a monomer composition for synthesizing recycled plastic, a recycled plastic and a molded product using the preparation method of a monomer composition for synthesizing recycled plastic, or the preparation device of a monomer composition for synthesizing recycled plastic.

**[Technical Solution]**

In order to achieve the above object, provided herein is a preparation method of a monomer composition for synthesizing recycled plastic, the method comprising the steps of: subjecting a polycarbonate-based resin to a depolymerization reaction in the presence of an alcohol; monitoring a Raman spectrum of an aromatic diol compound obtained from the depolymerization reaction or alcohol in a reactor through which depolymerization reaction proceeds; and

recovering the aromatic diol compound.

Also provided herein is a preparation device of a monomer composition for synthesizing recycled plastic, comprising: a reactor that subjects a polycarbonate-based resin to a depolymerization reaction in the presence of an alcohol; an in-line analyzer that is inserted in the reactor to monitor a Raman spectrum of an aromatic diol compound or alcohol obtained from the depolymerization reaction; and a recovery unit that recovers an aromatic diol compound obtained from the depolymerization reaction.

Further provided herein is a monomer composition for synthesizing recycled plastic, comprising an aromatic diol compound obtained by the preparation method of a monomer composition for synthesizing recycled plastic or the preparation device of a monomer composition for synthesizing recycled plastic.

Further provided herein is a recycled plastic comprising a reaction product of the monomer composition for synthesizing recycled plastic and a comonomer.

Further provided herein is a molded product comprising the recycled plastic.

Below, a preparation method of a monomer composition for synthesizing recycled plastic, a preparation device of a monomer composition for synthesizing recycled plastic, and a monomer composition for synthesizing recycled plastic, a recycled plastic and a molded product using the same according to specific embodiments of the present disclosure will be described in more detail.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

The 'pH' as used herein means a hydrogen ion concentration (pH), which is a numerical value indicating the acidity and alkalinity of a material. The PH can be determined from a value expressed by taking the reciprocal of the logarithmic dissociation concentration of hydrogen ions, and is used as a measure of the strength of acids and bases of a material.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present disclosure.

## 1. Preparation Method of Monomer Composition for Synthesizing Recycled Plastic

According to one embodiment of the present disclosure, there can be provided a preparation method of a monomer composition for synthesizing recycled plastic, the method comprising the steps of: subjecting a polycarbonate-based resin to a depolymerization reaction in the presence of an alcohol; monitoring a Raman spectrum of an aromatic diol compound obtained from the depolymerization reaction or alcohol in a reactor through which depolymerization reaction proceeds; and recovering the aromatic diol compound.

The present inventors have found through experiments that similarly to the method for preparing a monomer composition for recycling plastic synthesis according to the one embodiment, a Raman spectrum of an aromatic diol compound or alcohol obtained from the depolymerization reaction is monitored in a reactor in which depolymerization reaction proceeds in the process of recycling a polycarbonate-based resin by a chemical decomposition, so that the depolymerization reaction can be terminated at an optimal point, and thus, a high-purity aromatic diol compound can be recovered in a high yield, and completed the present disclosure.

In particular, conventionally, since the conversion rate of the final product is analyzed via HPLC or NMR analysis, or samples are collected and analyzed at any point during the reaction, there was a limitation in that it was difficult to find an optimal reaction end point for ensuring the aromatic diol compound in high purity and high yield, which is the substance to be recovered.

Meanwhile, according to the present disclosure, in the process of recycling a polycarbonate-based resin by a chemical decomposition, a Raman spectrum of the aromatic diol compound or alcohol obtained from the depolymerization reaction is secured in real time in the process of proceeding a depolymerization reaction through an analysis apparatus installed in a reactor in which the depolymerization reaction proceeds, and the approximate aromatic diol compound or alcohol can be determined by analyzing the spectrum, and thus the optimal reaction end point can be found more accurately. In addition, by minimizing the generation of reaction by-products while monitoring in real time the possibility of the generation of such by-products, it is possible to achieve the effect of recovering aromatic diol compounds with high purity and high yield.

Specifically, the preparation method of the monomer composition for synthesizing recycled plastics of the one embodiment may comprise a step of subjecting a polycarbonate-based resin to a depolymerization reaction in the presence of an alcohol.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate precursor. When it contains one carbonate repeating unit obtained by using only one type of aromatic diol compound and one type of carbonate-based compound, a homopolymer can be synthesized. In addition, when one type of aromatic diol compound and two or more types of carbonate-based compounds are used as the monomer, or two or more types of aromatic diol compounds and one type of carbonate-based compound are used, or one or more types of other diols is used in addition to the one type of aromatic diol compound and the one type of carbonate-based compound to contain two or more types of carbonates, a copolymer can be synthesized. The homopolymer or copolymer can include all of low-molecular compounds, oligomers, and polymers depending on the molecular weight range.

The polycarbonate-based resin can be applied regardless of various forms and types, such as a novel polycarbonate-based resin produced through synthesis, a recycled polycarbonate-based resin produced through a regeneration process, or polycarbonate-based resin waste.

However, if necessary, before proceeding a depolymerization reaction of the polycarbonate-based resin, a pretreatment step of the polycarbonate-based resin is carried out, thereby capable of increasing the efficiency of the process of recovering the aromatic diol compound and the carbonate precursor. Examples of the pretreatment step may include washing, drying, grinding, glycol decomposition, and the like. The specific method of each pretreatment step is not limited, and various methods widely used in the process of recovering the aromatic diol compound and the carbonate-based compound from the polycarbonate-based resin can be applied without limitation.

During the depolymerization reaction of the polycarbonate-based resin, the depolymerization reaction may be carried out under acidic, neutral or basic conditions, and particularly, the depolymerization reaction may be carried out under basic (alkali) conditions. The type of the base is not particularly limited, and examples thereof include sodium hydroxide (NaOH) or potassium hydroxide (KOH). The base is a base catalyst acting as a catalyst, and has the economic advantages over organic catalysts, which are mainly used under mild conditions. More specifically, during the depolymerization reaction of the polycarbonate-based resin, the depolymerization reaction may proceed within a pH range of more than 8 and less than 12.

During the depolymerization reaction of the polycarbonate-based resin, the depolymerization reaction may be carried out by reacting a base in an amount of 0.5 moles or less, or 0.4 moles or less, or 0.3 moles or less, or 0.1 moles or more, or 0.2 moles or more, or 0.1 moles to 0.5 moles, or 0.1 moles to 0.4 moles, or 0.1 moles to 0.3 moles, or 0.2 moles to 0.5 moles, or 0.2 moles to 0.4 moles, or 0.2 moles to 0.3 moles relative to 1 mole of polycarbonate-based resin. When the polycarbonate-based resin is reacted with a base in an amount of more than 0.5 moles relative to 1 mole of the polycarbonate-based resin during depolymerization of the polycarbonate-based resin, there is a limit that impurities increase due to the effect of increasing the amount of alkali salt generated, so the purity of the target recovery material is reduced, and the economic efficiency of the catalytic reaction is reduced.

Further, the depolymerization reaction of the polycarbonate-based resin can be carried out in the presence of an alcohol. The present disclosure can stably obtain bisphenol A, which is a high-purity monomer, by decomposing a polycarbonate-based resin with an alcohol, and has the advantage that a carbonate-based compound such as dialkyl carbonate having a high added value can be further obtained as a reaction by-product.

The number of hydroxy groups in the alcohol is not particularly limited, but may include, for example, monohydric alcohol. The monohydric alcohol is a compound having one hydroxy group in the molecule.

Further, the type of monovalent organic functional group bonded to the hydroxy group in the monohydric alcohol is not particularly limited, but may include an organic functional group having 1 to 10 carbon atoms, or 1 to 6 carbon atoms, such as an alkyl group, a cycloalkyl group, an aryl group, a heteroaryl group, or a combination of two or more thereof.

The alcohol may be one type of single compound or a mixture of two or more types. Specific examples of the alcohol are not particularly limited, but examples thereof include ethanol, methanol, phenol, or mixtures thereof.

The content of the alcohol may be 5 moles to 15 moles, or 8 moles to 13 moles relative to 1 mole of the polycarbonate-based resin. Since the alcohol has good solubility in bisphenol A, alcohol within the above range should be essentially contained. When the content of the alcohol is excessively reduced to less than 5 moles relative to 1 mole of the polycarbonate-based resin, it is difficult to sufficiently progress the alcohol decomposition of polycarbonate-based resin. On the other hand, when the content of alcohol is excessively increased to more than 15 moles relative to 1 mole of the polycarbonate-based resin, the economics of the process can be reduced due to excessive use of alcohol.

The solvent in which the depolymerization reaction of the polycarbonate-based resin proceeds may include at least one organic solvent selected from the group consisting of tetrahydrofuran, toluene, methylene chloride, chloroform, dimethyl carbonate, ethylmethyl carbonate, diethyl carbonate, and dipropyl carbonate.

The organic solvent may include tetrahydrofuran, toluene, methylene chloride, chloroform, dimethyl carbonate, ethylmethyl carbonate, diethyl carbonate, dipropyl carbonate, or a mixture of two or more thereof.

More preferably, methylene chloride can be used as the organic solvent. When methylene chloride is used as the organic solvent, there is an advantage that the dissolution properties in polycarbonate can be improved and the reactivity can be enhanced.

The content of the organic solvent may be 16 moles to 20 moles, or 16 moles to 18 moles relative to 1 mole of the polycarbonate-based resin. In addition, the content of the organic solvent may be 1.5 moles to 2 moles relative to 1 mole of alcohol. By mixing the polycarbonate-based resin, alcohol, and an organic solvent within the above range, there is an advantage that the depolymerization reaction of the polymer can proceed at a desired level.

Meanwhile, the temperature at which the depolymerization reaction of the polycarbonate-based resin proceeds is not particularly limited, but for example, the reaction may proceed at a temperature of 20°C to 100°C, or 50°C to 70°C. In addition, the depolymerization reaction of the polycarbonate-based resin may proceed for 1 hour to 30 hours, or 4 hours to 6 hours.

Specifically, the conditions are mild process conditions relative to the conventional pressurizing/high temperature process, and by performing stirring under the above conditions, the process can be performed in a mild process as compared to the pressurizing/high temperature process. In particular, when stirring at 50°C to 70°C for 4 to 6 hours, there is an advantage of obtaining the most efficient results in terms of reproducibility and stability.

That is, according to the present disclosure, as the type and mixing amount of the mixed solvent and the type and content of the base catalyst is adjusted without using an organic catalyst, there is the advantage that a high-purity aromatic diol compound (e.g., bisphenol A) can be obtained under mild conditions without using a pressure/high temperature process, and ethanol is used and thus, a diethyl carbonate can be obtained as by-products.

Meanwhile, during the depolymerization reaction of the polycarbonate-based resin, an antioxidant can be added to the reaction solution. As the antioxidant is added, the aromatic diol compound recovered through recycling by chemical decomposition of the polycarbonate-based resin can satisfy the low color coordinate b* value at a color level equivalent to that of reagents commercially sold or used for a PC polymerization.

Specific examples of the antioxidant are not particularly limited, and various antioxidants that have been widely used in the prior art can be applied without limitation. However, one example thereof include sodium hyposulfite, sodium sulfite, erythorbic acid, dibutylhydroxytoluene, butylhydroxyanisole, $\alpha$-tocopherol, tocopherol acetate, L-ascorbic acid and salts thereof, L-ascorbic acid palmitate, L-ascorbic acid stearate, triamyl gallate, propyl gallate or ethylenediamine tetraacetic acid disodium salt(EDTA), sodium pyrophosphate, sodium metaphosphate, or a mixture of two or more thereof.

The specific addition amount of the antioxidant is also not particularly limited, but as an example, the antioxidant can be added at a level that does not affect the physical properties of the monomer composition for synthesizing recycled plastic within the range of 0.1% by weight to 5 % by weight, or 0.1 % by weight to 1 % by weight based on the total weight of the reaction solution.

In addition, during the depolymerization reaction of the polycarbonate-based resin, the depolymerization can be carried out under a nitrogen atmosphere.

More specifically, the step of subjecting a polycarbonate-based resin to a depolymerization reaction may comprise a first step of dissolving the polycarbonate-based resin in an organic solvent; and a second step of adding a catalyst solution containing an alcohol, a base and an antioxidant. In the first and second steps, the details of the alcohol, organic solvent, base, antioxidant and polycarbonate-based resin are the same as described above.

Meanwhile, the preparation method of a monomer composition for synthesizing recycled plastic according to the one embodiment may include a step of monitoring a Raman spectrum of an aromatic diol compound obtained from the depolymerization reaction or alcohol in a reactor through which depolymerization reaction proceeds. The Raman spectrum of the aromatic diol compound or alcohol obtained from the depolymerization reaction may be monitored in real time during the progress of a depolymerization reaction through an analysis apparatus installed in a reactor in which the depolymerization reaction proceeds, and thus the optimal reaction end point can be found more accurately. In addition, as the generation of reaction by-products can be minimized by monitoring in real time the possibility of the generation of such by-products, it is possible to recover aromatic diol compounds in high purity and high yield.

In the step of monitoring a Raman spectrum of an aromatic diol compound obtained from the depolymerization reaction or alcohol in a reactor through which depolymerization reaction proceeds, the Raman spectrum is data obtained by Raman analysis. For specific details of the Raman analysis, various conventionally known analysis methods, analysis apparatuses and analysis conditions can be applied without limitation. However, as an example, MarqMetrix All-in-one apparatus can be used.

In particular, the step of monitoring a Raman spectrum of an aromatic diol compound obtained from the depolymerization reaction or alcohol in a reactor through which depolymerization reaction proceeds can proceed via a Raman probe inserted into the reactor. More specifically, in one embodiment, the in-line probe, which is a Raman probe inserted into a reactor, can be used to analyze the real-time chemical component change in the solid/liquid reaction solution in a non-destructive manner.

Accordingly, the Raman spectrum of an aromatic diol compound (e.g., bisphenol A) generated through the depolymerization reaction of a polycarbonate-based resin or an alcohol (e.g., ethanol) participating in the reaction can be confirmed in real time through an in-line Raman probe, hereby making it possible to indirectly predict the real-time change in the concentration and content of the aromatic diol compound (or alcohol). More specifically, a PLS (partial least squares) prediction model can be secured through correlation between Raman spectrum data and the concentration and content values secured through HPLC analysis.

The Raman measuring device may be manufactured to withstand the reaction pressure, and then inserted into a reactor in which a depolymerization reaction proceeds to collect reaction information in real time. One end of the Raman probe can be in contact with the reactant of the depolymerization reaction, and the other end can be connected to a computer so that the obtained Raman spectrum can be analyzed in real time. For specific details of the Raman probe, any conventionally known contents can be applied without limitation. However, as an example, a BallProbe can be used.

In a specific example of monitoring the Raman spectrum, the step of monitoring a Raman spectrum of the aromatic diol compound or alcohol obtained from the depolymerization reaction in the reactor in which the depolymerization reaction proceeds may comprise a step of measuring an integral ratio for an aromatic diol compound according to the following Mathematical Formula 1:

Integral ratio for aromatic diol compound = { Integral value of the region from 791.2 cm$^{-1}$ to 861.5 cm$^{-1}$ on the Raman spectrum / (Integral value of the region from 663.4 cm$^{-1}$ to 785.1 cm$^{-1}$ on the Raman spectrum + Integral value of the region from 234.8 cm$^{-1}$ to 337.1 cm$^{-1}$ on the Raman spectrum)}          [Mathematical Formula 1]

in the Mathematical Formula 1, the region from 791.2 cm$^{-1}$ to 861.5 cm$^{-1}$ on the Raman spectrum is the spectrum of an aromatic diol compound, and
the region from 663.4 cm$^{-1}$ to 785.1 cm$^{-1}$ and from 234.8 cm$^{-1}$ to 337.1 cm$^{-1}$ on the Raman spectrum is the spectrum of a reaction solvent.

In Mathematical Formula 1, since the methylene chloride component used as the depolymerization reaction solvent does not participate in the depolymerization reaction, the integral value of Raman band of the reaction solvent is set as the standard value in the denominator, and then the integral value of Raman band of the aromatic diol compound is taken in the numerator to calculate the relative integral value.

The integral ratio of the aromatic diol compound according to Mathematical Formula 1 has a value between 0 and 0.15, which starts from 0 to 0.06 at the beginning of the depolymerization reaction, rapidly increases to 0.1 or more, or 0.1 to 0.11 within 30 minutes after the start of the reaction, and shows a tendency to converge to a value between 0.11 and 0.15, or 0.11 and 0.13, or 0.110 and 0.125, or 0.115 and 0.125, or 0.115 and 0.122, or 0.12 and 0.122 after 30 minutes.

Based on this understanding of the tendency, it was confirmed that when the integral ratio for aromatic diol compound according to Mathematical Formula 1 is 0.115 to 0.125, or 0.115 to 0.122, or 0.12 to 0.122, terminating the depolymerization reaction enables the recovery of aromatic diol compounds with high purity and high yield while securing process efficiency. When the integral ratio for aromatic diol compound according to Mathematical Formula 1 decreases to less than 0.115, it is difficult to sufficiently secure the yield of the aromatic diol compound. On the other hand, when the integral ratio for aromatic diol compound according to Mathematical Formula 1 increases to more than 0.125, impurities other than the aromatic diol compound (for example, impurities derived from the aromatic diol compound) increase, which makes it difficult to sufficiently secure the purity of the aromatic diol compound, and there is a limitation that the process efficiency decreases as the reaction proceeds for too long

Meanwhile, the step of monitoring a Raman spectrum of an aromatic diol compound obtained from the depolymerization reaction or alcohol in a reactor through which depolymerization reaction proceeds can proceed repeatedly at intervals of 5 minutes or less. Specifically, the step of monitoring a Raman spectrum of an aromatic diol compound obtained from the depolymerization reaction or alcohol in a reactor through which depolymerization reaction proceeds can proceed repeatedly at intervals of 5 minutes or less, or 4 minutes or less, or 3 minutes or less during the progress of a depolymerization reaction.

As the interval for monitoring the Raman spectrum of the aromatic diol compound or alcohol obtained from the depolymerization reaction is shorter, the optimal reaction end point can be found more accurately and the generation of reaction byproducts can be minimized.

In addition, in another specific example of monitoring the Raman spectrum, the step of monitoring a Raman spectrum of the aromatic diol compound or alcohol obtained from the depolymerization reaction in the reactor in which the depolymerization reaction proceeds may comprise a step of measuring an integral ratio for an alcohol according to the following Mathematical Formula 2:

Integration ratio for alcohol = {Integral value of the region from 849.7 cm$^{-1}$ to 910 cm$^{-1}$ on the Raman spectrum / (Integral value of the region from 663.4 cm$^{-1}$ to 785.1 cm$^{-1}$ on the Raman spectrum + Integral value of the region from 234.8 cm$^{-1}$ to 337.1 cm$^{-1}$ on the Raman spectrum)}

[Mathematical Formula 2]

in the Mathematical Formula 2, the region from 849.7 cm$^{-1}$ to 910 cm$^{-1}$ on the Raman spectrum is the spectrum of an alcohol, and the region from 663.4 cm$^{-1}$ to 785.1 cm$^{-1}$ and from 234.8 cm$^{-1}$ to 337.1 cm$^{-1}$ on the Raman spectrum is the spectrum of a reaction solvent.

In Mathematical Formula 2, since the methylene chloride component used as the depolymerization reaction solvent does not participate in the depolymerization reaction, the integral value of Raman band of the reaction solvent is set as the standard value in the denominator, and then the integral value of Raman band of the alcohol is taken in the numerator to calculate the relative integral value.

The integral ratio of the alcohol according to Mathematical Formula 2 has a value between 0 and 1.1, which starts from 0.15 or more, or 0.15 to 1.1 at the beginning of the depolymerization reaction, then rapidly decreases to 0.11 or less, or 0.1 to 0.11 within 30 minutes after the start of the reaction, and then tends to converge to a value between 0.09 and 0.1, or 0.095 and 0.1 after 30 minutes.

Based on this understanding of the tendency, it was confirmed that when the integral ratio for alcohol according to Mathematical Formula 2 is 0.09 to 0.1, or 0.095 to 0.1, terminating the depolymerization reaction enables the recovery of aromatic diol compounds with high purity and high yield while securing process efficiency. When the integral ratio for alcohol according to Mathematical Formula 2 increases to more than 0.1, it is difficult to sufficiently secure the yield of the aromatic diol compound. On the other hand, when the integral ratio for alcohol according to Mathematical Formula 2 decreases to less than 0.09, impurities other than the aromatic diol compound (for example, impurities derived from the aromatic diol compound) increase, which makes it difficult to sufficiently secure the purity of the aromatic diol compound, and there is a limitation that the process efficiency decreases as the reaction proceeds for too long

Furthermore, the step of monitoring a Raman spectrum of an aromatic diol compound obtained from the depolymerization reaction or alcohol in a reactor through which depolymerization reaction proceeds can proceed repeatedly at intervals of 5 minutes or less. Specifically, the step of monitoring a Raman spectrum of an aromatic diol compound obtained from the depolymerization reaction or alcohol in a reactor through which depolymerization reaction proceeds can proceed repeatedly at intervals of 5 minutes or less, or 4 minutes or less, or 3 minutes or less during the progress of a depolymerization reaction. As the interval for monitoring the Raman spectrum of the aromatic diol compound or alcohol obtained from the depolymerization reaction is shorter, the optimal reaction end point can be found more accurately and the generation of reaction byproducts can be minimized.

Meanwhile, the step of monitoring a Raman spectrum of an aromatic diol compound obtained from the depolymerization reaction or alcohol in a reactor through which depolymerization reaction proceeds may further comprise a step of monitoring the Raman spectrum of impurities derived from an aromatic diol compound produced by the depolymerization reaction.

The impurities derived from an aromatic diol compound are by-products generated as the depolymerization reaction proceeds excessively, and there is a problem in that they reduce the purity of bisphenol A, which is an aromatic diol compound to be recovered. Therefore, the Raman spectrum of the impurities derived from an aromatic diol compound produced by the depolymerization reaction is monitored in real time, so that the occurrence of reaction by-products is monitored in real time, and by-products are minimized, thereby making it possible to recover aromatic diol compounds with high purity and high yield.

The impurities derived from an aromatic diol compound refer to all substances except for the aromatic diol compound, which is the main substance to be recovered in the present disclosure, and the specific type thereof is not particularly limited, but an example thereof may be p-tert-butylphenol.

Specifically, the step of monitoring the Raman spectrum of impurities derived from an aromatic diol compound produced by the depolymerization reaction may comprise a step of measuring the integration ratio for impurities derived from an aromatic diol compound according to the following Mathematical Formula 3:

Integration ratio for impurities derived from aromatic diol compound = {Integral value of the region from 800 cm$^{-1}$ to 850 cm$^{-1}$ on the Raman spectrum / (Integral value of the region from 663.4 cm$^{-1}$ to 785.1 cm$^{-1}$ on the Raman spectrum + Integral value of the region from 234.8 cm$^{-1}$ to 337.1 cm$^{-1}$ on the Raman spectrum)}

[Mathematical Formula 3]

in the Mathematical Formula 3, the region from 800 cm$^{-1}$ to 850 cm$^{-1}$ on the Raman spectrum is the spectrum of impurities derived from aromatic diol compound, and the region from 663.4 cm$^{-1}$ to 785.1 cm$^{-1}$ and from 234.8 cm$^{-1}$ to 337.1 cm$^{-1}$ on the Raman spectrum is the spectrum of a reaction solvent.

In Mathematical Formula 3, since the methylene chloride component used as the depolymerization reaction solvent does not participate in the depolymerization reaction, the Raman band integral value of the reaction solvent is set as the standard value in the denominator, and then the Raman band integral value of the impurities derived from aromatic diol compound is taken in the numerator to calculate the relative integral value.

When the integral ratio of the impurities derived from aromatic diol compound according to Mathematical Formula 3 exceeds 0.12, terminating the depolymerization reaction enables the recovery of aromatic diol compounds with high purity and high yield while securing process efficiency. When the integral ratio for the impurities derived from aromatic diol compound according to Mathematical Formula 3 increases to more than 0.12, impurities other than the aromatic diol compound (for example, impurities derived from the aromatic diol compound) increase, which makes it difficult to sufficiently secure the purity of the aromatic diol compound, and there is a limitation that the process efficiency decreases as the reaction proceeds for too long

Meanwhile, the preparation method of a monomer composition for synthesizing recycled plastic may comprise a step of recovering an aromatic diol compound obtained from the depolymerization reaction. Specific examples of the aromatic diol compound include bis(4-hydroxyphenyl)methane, bis(4-hydroxyphenyl)ether, bis(4-hydroxyphenyl)sulfone, bis(4-hydroxyphenyl)sulfoxide, bis(4-hydroxyphenyl)sulfide, bis(4-hydroxyphenyl)ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or a mixture of two or more thereof, and the like. Preferably, the aromatic diol compound of the monomer composition for synthesizing recycled plastics of the one embodiment may be 2,2-bis(4-hydroxyphenyl)propane (bisphenol A).

Specifically, the step of recovering an aromatic diol compound obtained from the depolymerization reaction may further comprise a step of adding an acid so that the pH of the depolymerization reaction product becomes 2 to 8. A strong acid can be used as the acid, and examples thereof include hydrochloric acid (HCl).

In the step of adding an acid so that the pH of the depolymerization reaction product becomes 2 to 8, the salt of the aromatic diol compound included in the depolymerization reaction product may be converted into the aromatic diol compound. As the depolymerization reaction proceeds under basic conditions, the resulting aromatic diol compound exists in the form of a salt upon reaction with a base, and thus has hydrophilicity. Thereby, by adding an acid, the salt of the aromatic diol compound contained in the depolymerization reaction product can be converted into the aromatic diol compound, thereby inducing to have hydrophobicity.

Thus, a layer divided into a water layer containing impurities and an organic solvent layer containing an aromatic diol compound and a carbonate precursor can be formed in the step of adding water to remove impurities after adding the acid, which will be described later. Since the aromatic diol compound and the carbonate precursor have hydrophobicity, they may be included in an organic solvent layer among water and an organic solvent, and various water-soluble impurities can be included in the water layer. Thereby, the main products, i.e., aromatic diol compounds and impurities, can be easily separated only by a simple step of changing the pH.

Meanwhile, the step of adding an acid so that the pH of the depolymerization product is 2 to 8 may further comprise a step of adding water. Thereby, a layer divided into a water layer containing impurities and an organic solvent layer containing an aromatic diol compound and a carbonate precursor can be formed in the step of removing impurities which will be described later.

The order of the step of adding the acid and the step of adding water is not particularly limited, and addition of water after addition of acid, addition of acid after addition of water, or simultaneous addition of water and acid are possible.

Meanwhile, the step of recovering an aromatic diol compound obtained from the depolymerization reaction may comprise a step of removing impurities after the addition of acid. Therefore, the impurities are materials having hydrophilicity, and examples thereof may include a salt compound, an ionic compound, an acid compound, and the like.

As described above, after the step of adding an acid so that the pH of the depolymerization reaction product is 2 to 8, as the step of removing impurities from the depolymerization reaction product after addition of the acid progresses, the depolymerization reaction product forms a layer divided into a water layer containing impurities, and an organic solvent layer containing an aromatic diol compound and a carbonate precursor, so that the water layer containing impurities can be separated and removed.

In the step of removing impurities from the depolymerization reaction product after addition of the acid, the water layer can be separated from the organic layer to remove impurities included in the water layer. Specific separation conditions for separating the water layer from the organic layer are not particularly limited. As for the specific separation devices and methods, various well-known purification techniques can be applied without limitation. However, as an example, a drain device can be used.

Meanwhile, the step of recovering an aromatic diol compound obtained from the depolymerization reaction may comprise a step of separating the carbonate precursor from the depolymerization reaction product. Thus, the separated carbonate precursor may include diethyl carbonate.

The step of separating the carbonate precursor from the depolymerization reaction product may include a reduced pressure distillation step of the depolymerization product. Examples of the reduced pressure distillation conditions are not particularly limited, but in a specific example, the product of the depolymerization reaction of the polycarbonate-based resin is pressurized under a pressure of 200 mbar to 300 mbar and a temperature of 20°C to 30°C, and then decompressed under a pressure of 10 mbar to 50 mbar and a temperature of 20°C to 30°C and subjected to a low-temperature distillation.

The separated carbonate precursor can be recycled without a separate purification process, or can be recycled through separation and purification such as conventional extraction, adsorption, and drying, if necessary. Specific purification conditions are not particularly limited. As for the specific purification devices and methods, various well-known purification techniques can be applied without limitation.

Meanwhile, the step of recovering an aromatic diol compound obtained from the depolymerization reaction may further comprise a step of purifying the depolymerization reaction product from which the carbonate precursor has been separated, after the step of separating the carbonate precursor from the depolymerization reaction product.

Specifically, the step of purifying the depolymerization reaction product from which the carbonate precursor has been separated may comprise a step of washing the depolymerization reaction product from which the carbonate precursor has been separated. In addition, the step of purifying the depolymerization reaction product from which the carbonate precursor has been separated may include an adsorption purification step of the depolymerization reaction product from which the carbonate precursor has been separated. Further, the step of purifying the depolymerization reaction product from which the carbonate precursor has been separated may include a recrystallization step of the depolymerization reaction product from which the carbonate precursor has been separated.

The order of the washing step; the adsorption purification step; or the recrystallization step is not particularly limited, and it is irrelevant to proceed in any order, but for example, the washing step; the adsorption purification step; and the recrystallization step can proceed in this order. The washing step; the adsorption purification step; and the recrystallization step can proceed repeatedly at least once or more. As for the specific washing, adsorption and recrystallization apparatuses and methods, various well-known purification techniques can be applied without limitation.

Specifically, in the washing step of the depolymerization reaction product from which the carbonate precursor has been separated, the depolymerization reaction product from which the carbonate precursor has been separated may contain an aromatic diol compound. However, since various impurities remain during the recovery process of obtaining the aromatic diol compound, washing can proceed in order to sufficiently remove these impurities and secure a high-purity aromatic diol compound.

Specifically, the washing step may include a step of washing with a solvent at a temperature of 10°C or more and 30°C or less, or 20°C or more and 30°C or less. The temperature condition means the temperature inside the washing container at which washing with a solvent is performed. In order to maintain a high temperature deviating from a room temperature, various heating devices can be applied without limitation.

The solvent used in the washing step may include one of water, alcohol, and an organic solvent. As the organic solvent, tetrahydrofuran, toluene, methylene chloride, chloroform, dimethyl carbonate, ethylmethyl carbonate, diethyl carbonate, dipropyl carbonate, or a mixture of two or more thereof can be used.

The solvent used in the washing step can be used in a weight ratio of 1 part by weight or more and 30 parts by weight or less, or 1 part by weight or more and 10 parts by weight or less, based on 1 part by weight of the polycarbonate-based resin used in the depolymerization reaction.

More specifically, the solvent in the step of washing with a solvent at a temperature of 10°C or more and 30°C or less may be an organic solvent. Preferably, methylene chloride can be used as the organic solvent. At this time, the organic solvent can be used in an amount of 1 part by weight or more and 10 parts by weight or less, based on 1 part by weight of the polycarbonate-based resin.

Further, the adsorption purification step of the depolymerization reaction product from which the carbonate precursor has been separated may comprise a step of adding an adsorbent to the depolymerization reaction product from which the carbonate precursor has been separated to perform an adsorption purification and then removing the adsorbent. **In** the step of adding an adsorbent to the depolymerization reaction product from which the carbonate precursor has been separated to perform an adsorption purification and then removing the adsorbent, an adsorbent can be brought into contact with the depolymerization reaction product.

Examples of the adsorbent that can be used include activated carbon, charcoal, or a mixture thereof. The activated carbon is a black carbon material having micropores produced by subjecting a raw material to a carbonization process at about 500°C and an activated carbon process at about 900°C, and examples thereof are not particularly limited, but for example, various activated carbons such as plant-based, coal-based, petroleum-based, waste-based activated carbons can be applied without limitation depending on the type of raw material.

In more specific examples, the plant-based activated carbon may include coconut activated carbon, wood activated carbon, and sawdust activated carbon. Further, the coal-based activated carbon may include lignite activated carbon, bituminous coal activated carbon, and anthracite activated carbon. Further, the petroleum-based activated carbon may include petroleum coke activated carbon and oil carbon activated carbon. Further, the waste activated carbon may include synthetic resin activated carbon and pulp activated carbon.

The adsorbent may include at least one activated carbon selected from the group consisting of plant-based activated carbon, coal-based activated carbon, petroleum-based activated carbon, and waste-based activated carbon. That is, the adsorbent may include plant-based activated carbon, coal-based activated carbon, petroleum-based activated carbon, waste-based activated carbon, or a mixture of two or more thereof.

More specifically, the adsorbent may include at least one activated carbon selected from the group consisting of coconut activated carbon, lignite activated carbon, anthracite activated carbon, and bituminous coal activated carbon. That is, the adsorbent may include coconut activated carbon, lignite activated carbon, anthracite activated carbon, bituminous coal activated carbon, or a mixture of two or more thereof.

Adsorption purification conditions by the adsorbent are not particularly limited, and various well-known adsorption purification conditions can be used without limitation. However, in one example, the addition amount of the adsorbent may be 40% by weight to 60% by weight relative to the polycarbonate-based resin, and the adsorption time may be 1 hour to 5 hours, and the adsorption method may be a stirring adsorption or an adsorption tower for Lab.

If necessary, the method may further include a step of adding a solvent to the depolymerization reaction product from which the carbonate precursor has been separated, prior to the step of adding an adsorbent to the depolymerization reaction product from which the carbonate precursor has been separated to perform an adsorption purification and then removing the adsorbent. Examples of the solvent include ethanol, and the ethanol may be added in a ratio of 1 mole to 20 moles, or 10 moles to 20 moles, or 15 moles to 20 moles relative to 1 mole of the polycarbonate-based resin. Aromatic diol compound crystals contained in the depolymerization reaction product from which the carbonate precursor has been separated can be redissolved in a solvent through the step of adding a solvent to the depolymerization reaction product in which the carbonate precursor has been separated.

Meanwhile, in the recrystallization step of the depolymerization reaction product from which the carbonate precursor has been separated, a high-purity aromatic diol compound can be secured by sufficiently removing various impurities contained in the depolymerization product from which the carbonate precursor has been separated.

Specifically, the recrystallization step may include a step of adding water to the depolymerization reaction product from which the carbonate precursor has been separated to perform recrystallization. Through the step of adding water to the depolymerization reaction product from which the carbonate precursor has been separated to perform recrystallization, the solubility of the aromatic diol compound or its salt contained in the depolymerization reaction product is increased, and thus, crystals, or impurities interposed between crystals can be dissolved with a solvent to the maximum, and further, since the dissolved aromatic diol compound has poor solubility relative to impurities, it can be easily precipitated into aromatic diol compound crystals through the difference in solubility when the temperature is lowered subsequently.

More specifically, in the step of adding water to the depolymerization reaction product from which the carbonate precursor has been separated to perform recrystallization, 200 moles to 400 moles, or 250 moles to 350 moles of water can be used with respect to 1 mole of the polycarbonate-based resin. When the water is used in an excessively small amount, the temperature for dissolving the aromatic diol compound contained in the depolymerization reaction product from which the carbonate precursor has been separated becomes too high, which thus deteriorates in the process efficiency, and it is no easy to remove impurities through recrystallization. On the other hand, when water is used in an excessively large amount, the solubility of the aromatic diol compound contained in the depolymerization reaction product from which the carbonate precursor has been separated becomes too high, and thus, the yield of the aromatic diol compound recovered after recrystallization is reduced, and the process efficiency can be reduced due to the use of large amounts of solvent.

If necessary, after proceeding the recrystallization step of the depolymerization reaction product from which the carbonate precursor has been separated, a step of removing residual impurities through filtration or adsorption can be further performed.

In addition, if necessary, after the recrystallization step, the method may further include a drying step. The remaining solvent can be removed by the drying, and the specific drying conditions are not particularly limited, but for example, the drying can be performed at a temperature of 10°C to 100°C, or 10°C to 50°C. As for the specific drying apparatuses and methods used in the drying, various well-known drying techniques can be applied without limitation.

**2. Preparation device of a monomer composition for synthesizing recycled plastic**

According to another embodiment of the invention, there can be provided a preparation device of a monomer composition for synthesizing recycled plastic, comprising: a reactor that subjects a polycarbonate-based resin to a depolymerization reaction in the presence of an alcohol; an in-line analyzer that is inserted in the reactor to monitor a Raman spectrum of an aromatic diol compound or alcohol obtained from the depolymerization reaction; and a recovery unit that recovers an aromatic diol compound obtained from the depolymerization reaction.

The details of subjecting a polycarbonate-based resin to a depolymerization reaction in the presence of an alcohol include all the contents described above in the one embodiment. The specific shape, material, and size of the reactor are not particularly limited, and various reactors that have been widely used in conventional polymer depolymerization reactions can be applied without limitation.

The details of being inserted in the reactor to monitor a Raman spectrum of an aromatic diol compound or alcohol obtained from the depolymerization reaction include all the contents described above in the one embodiment.

The in-line analyzer may include a Raman probe. The Raman probe may be manufactured to withstand the reaction pressure and then inserted into a reactor in which a depolymerization reaction proceeds to collect reaction information in real time. One end of the Raman probe can be in contact with the reactant of the depolymerization reaction, and the other end can be connected to a computer so that the obtained Raman spectrum can be analyzed in real time. Specifically, the values of Mathematical Formulas 1, 2 and 3 of the embodiments can be analyzed through the computer.

The in-line analyzer may include a detection unit that comes into contact with the reactants in the reactor. In addition, the preparation device may further include a computer that analyzes the Raman spectrum measured by the in-line analyzer. One end of the inline analyzer may come into contact with the reactants in the reactor, and the other end may be connected to a computer so that the obtained spectrum can be analyzed in real time.

The details of recovering the aromatic diol compound obtained from the depolymerization reaction in the recovery unit includes all the contents described above in the embodiments. The specific shape, material, and size of the recovery unit are not particularly limited, and various recovery apparatuses that have been widely used in conventional depolymerization reaction of a polycarbonate can be applied without limitation. However, for example, distillation apparatus, washing apparatus, filtration apparatus, recrystallization apparatus, etc. can be used in the recovery unit.

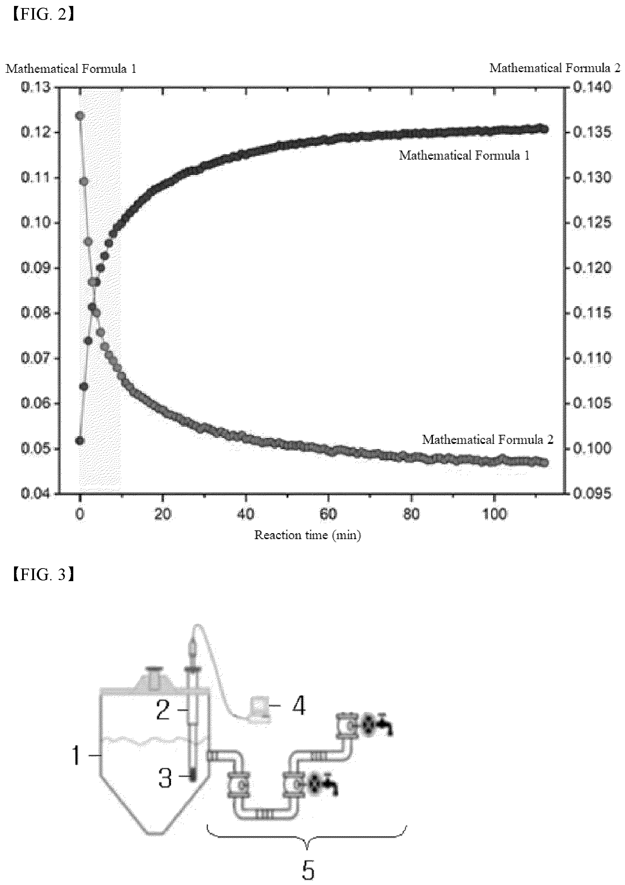

A specific example of the preparation device of a monomer composition for synthesizing recycled plastic according to another embodiment of the invention is not particularly limited, but as an example, referring to FIG. 3, it may include a reactor 1 that subjects a polycarbonate-based resin to a depolymerization reaction in the presence of an alcohol; an in-line analyzer 2 that is inserted in the reactor to monitor a Raman spectrum of an aromatic diol compound or alcohol obtained from the depolymerization reaction; and a recovery unit 5 that recovers an aromatic diol compound obtained from the depolymerization reaction. More specifically, the in-line analyzer may include a detection unit 3 that comes into contact with a reactant in the reactor. In addition, the preparation device may further include a computer 4 that analyzes a Raman spectrum measured by the inline analyzer.

### 3. Monomer composition for synthesizing recycled plastic

According to yet another embodiment of the invention, there can be provided a monomer composition for synthesizing recycled plastic, comprising an aromatic diol compound obtained by the preparation method of a monomer composition for synthesizing recycled plastic according to the one embodiment, or the preparation device of a monomer composition for synthesizing recycled plastic according to another embodiment.

That is, the monomer composition for recycling plastic synthesis of the other embodiments may be obtained by the preparation method of the monomer composition for recycling plastic synthesis of the one embodiment. The details of the preparation method of the monomer composition for recycling plastic synthesis of the one embodiment includes all the content described above in the one embodiment.

In addition, the monomer composition for synthesizing recycled plastic of the other embodiments may be obtained by the preparation device of the monomer composition for recycling plastic synthesis of the other embodiments. The details of the preparation device of the monomer composition for synthesizing recycled plastic of the other embodiments include all the contents described above in the one embodiment.

Specific examples of the aromatic diol compound include bis(4-hydroxyphenyl)methane, bis(4-hydroxyphenyl) ether, bis(4-hydroxyphenyl)sulfone, bis(4-hydroxyphenyl)sulfoxide, bis(4-hydroxyphenyl)sulfide, bis(4-hydroxyphenyl) ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or a mixture of two or more thereof, and the like. Preferably, the aromatic diol compound of the monomer composition for synthesizing recycled plastics of the one embodiment may be 2,2-bis(4-hydroxyphenyl)propane (bisphenol A).

The aromatic diol compound is characterized by being obtained from the preparation method of a monomer composition for synthesizing recycled plastic of the one embodiment, or the preparations device of a monomer composition for synthesizing recycled plastic of the other embodiment. That is, the aromatic diol compound is characterized by being recovered from the polycarbonate-based resin used in the recovery of the monomer composition for synthesizing recycled plastic. Therefore, apart from the recovery from the polycarbonate-based resin in order to prepare the monomer composition for synthesizing recycled plastics according to the other embodiments, the case where a novel aromatic diol compound is added from the outside is not included in the category of aromatic diol compound of the present disclosure.

Specifically, "being recovered from the polycarbonate-based resin" means being obtained through a depolymerization reaction of the polycarbonate-based resin. The depolymerization reaction can proceed under acidic, neutral or basic conditions, and particularly, the depolymerization reaction can proceed under basic (alkaline) conditions. Particularly, the depolymerization reaction can proceed preferably in the presence of an ethanol solvent, as will be described later.

## 4. Recycled Plastic

According to yet another embodiment of the invention, there can be provided a recycled plastic comprising a reaction product of the monomer composition for synthesizing a recycled plastic of the other embodiment and a comonomer.

The details of the monomer composition for synthesizing recycled plastic of the other embodiment includes all the contents described above in the other embodiment.

Examples corresponding to the recycled plastic are not particularly limited, and various plastics synthesized from aromatic diol compounds such as bisphenol A and a carbonate precursor such as dimethyl carbonate, diethyl carbonate, or ethylmethyl carbonate as a monomer can be applied without limitation, and a more specific example may be a polycarbonate-based resin.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate precursor. When it contains one type of carbonate repeating unit obtained by using only one type of aromatic diol compound and one type of carbonate-based compound, a homopolymer can be synthesized. **In** addition, when one type of aromatic diol compound and two or more types of carbonate-based compounds are used as the monomer, or two or more types of aromatic diol compounds and one type of carbonate-based compound are used, or one or more types of other diols is used in addition to the one type of aromatic diol compound and the one type of carbonate-based compound to contain two or more carbonates, a copolymer can be synthesized. The homopolymer or copolymer can include all of low-molecular compounds, oligomers, and polymers depending on the molecular weight range.

More specifically, in the recycled plastic containing the reaction product of the monomer composition for synthesizing the recycled plastic and the comonomer according to the one embodiment, a carbonate precursor can be used as the comonomer. Specific examples of the carbonate-based compound include phosgene, triphosgene, diphosgene, bromophosgene, dimethyl carbonate, diethyl carbonate, dibutyl carbonate, dicyclohexyl carbonate, diphenyl carbonate, ditolyl carbonate, bis(chlorophenyl)carbonate, m-cresyl carbonate, dinaphthyl carbonate, bis(diphenyl) carbonate or bishaloformate.

Examples of the reaction process of the monomer composition for synthesizing recycled plastic and the comonomer that synthesizes the polycarbonate-based resin are not particularly limited, and various well-known methods for preparing polycarbonate can be applied without limitation.

However, in one example of the method for preparing a polycarbonate, a method for preparing a polycarbonate comprising a step of polymerizing a composition containing a monomer composition for synthesizing recycled plastic and a comonomer can be used. At this time, the polymerization can be carried out by interfacial polymerization, and during interfacial polymerization, polymerization reaction is possible at normal pressure and low temperature, and the molecular weight is easy to control.

The polymerization temperature may be 0°C to 40°C, and the reaction time may be 10 minutes to 5 hours. In addition, the pH during the reaction may be maintained at 9 or more or 11 or more.

The solvent that can be used for the polymerization is not particularly limited as long as it is a solvent used for polymerization of polycarbonate in the art, and as an example, halogenated hydrocarbons such as methylene chloride and chlorobenzene can be used.

Moreover, the polymerization can be carried out in the presence of an acid binder. As the acid binder, an alkali metal hydroxide such as sodium hydroxide or potassium hydroxide, or an amine compound such as pyridine can be used.

Further, in order to adjust the molecular weight of the polycarbonate during the polymerization, polymerization can be carried out in the presence of a molecular weight modifier. An alkylphenol having 1 to 20 carbon atoms may be used as the molecular weight modifier, and specific examples thereof include p-tert-butylphenol, p-cumylphenol, decylphenol, dodecylphenol, tetradecylphenol, hexadecylphenol, octadecylphenol, eicosylphenol, docosylphenol or triacontylphenol. The molecular weight modifier can be added before, during or after the initiation of polymerization. The molecular weight modifier may be used in an amount of 0.01 to 10 parts by weight, or 0.1 to 6 parts by weight, based on 100 parts by weight of the aromatic diol compound, and a desired molecular weight can be obtained within this range.

In addition, in order to promote the polymerization reaction, a reaction accelerator such as a tertiary amine compound, a quaternary ammonium compound, or a quaternary phosphonium compound, including triethylamine, tetra-n-butylammonium bromide, or tetra-n-butylphosphonium bromide can be further used.

**5. Molded Product**

According to yet another embodiment of the invention, a molded article comprising the recycled plastic according to the other embodiment can be provided. The details of the recycled plastic includes all the contents described above in the other embodiments.

The molded article can be obtained by applying the recycled plastic to various known plastic molding methods without limitation. As an example of the molding method, injection molding, foam injection molding, blow molding, or extrusion molding may be mentioned.

Examples of the molded article are not particularly limited, and can be applied to various molded articles using plastic without limitation. Examples of the molded article include automobiles, electrical and electronic products, communication products, daily necessities, building materials, optical components, exterior materials, and the like.

The molded article may further include one or more additives selected from the group consisting of an antioxidant, a plasticizer, an antistatic agent, a nucleating agent, a flame retardant, a lubricant, an impact enhancer, an optical brightener, an ultraviolet absorber, a pigment and a dye, if necessary, in addition to the recycled plastic according to the other embodiments,

An example of the manufacturing method of the molded article may include a step of mixing the recycled plastic according to the other embodiment and an additive well using a mixer, extrusion-molding the mixture with an extruder to produce pellets, drying the pellets, and then injecting them with an injection molding machine.

**[Advantageous Effects]**

According to the present disclosure, there can be provided a preparation method of a monomer composition for synthesizing recycled plastic, a preparation device of a monomer composition for synthesizing recycled plastic, a monomer composition for synthesizing recycled plastic, a recycled plastic and a molded product using the same, wherein the process of recycling aromatic diol compounds through a chemical decomposition of a polycarbonate-based resin is monitored in real time to optimize reaction conditions and improve efficiency.

**[BRIEF DESCRIPTION OF THE DRAWINGS]**

FIG. 1 shows the Raman spectrum obtained in Examples.
FIG. 2 shows the integral ratio for the aromatic diol compound and the integral ratio for the alcohol obtained in Example 1.
FIG. 3 shows a schematic diagram of a preparation device of a monomer composition for synthesizing recycled plastic.

**[DETAILED DESCRIPTION OF THE EMBODIMENTS]**

Hereinafter, the present disclosure will be explained in detail with reference to the following examples. However, these examples are for illustrative purposes only, and the scope of the present disclosure is not limited thereto.

**<EXAMPLE and COMPARATIVE EXAMPLE: Preparation of recycled bisphenol A monomer composition>**

Example 1

(1. Decomposition step) 1 mol of waste polycarbonate(PC) was added to a reactor 1 and dissolved using methylene chloride(MC), and then foreign materials were filtered out. Then, 11 mol of ethanol (EtOH), 0.2 mol of sodium hydroxide(NaOH), and an antioxidant were added thereto and stirred at 60°C to perform a PC depolymerization reaction.

During the PC depolymerization reaction, an in-line analyzer 2, i.e., a Raman probe, was inserted into the reactor 1, and set so that a detection unit 3 was not disturbed due to the viscosity of the polycarbonate(PC) during stirring. The Raman spectrum was measured at regular intervals (about 3 minutes) for a total of 6 hours after the start of the reaction, and analyzed in real time using a computer 4 program, and the reaction was terminated at the optimal reaction point (BPA/MC Raman integral ratio = 0.12).

(2. pH adjustment) The product of the depolymerization reaction was cooled to 30°C or less, and then adjusted to have pH 8 by adding 10% hydrochloric acid (HCl) and water.

(3. Layer separation) After that, in a state in which the water layer and the methylene chloride (MC) layer were formed, the organic layer located on the lower side was recovered using a drain device located at the lower end of the reactor, and the water layer located on the upper side was discharged and then discarded.

(4. Distillation) After that, the recovered methylene chloride(MC) layer was moved to a recovery unit 5, and the by-product diethyl carbonate (DEC) was separated and recovered through low-temperature distillation reducing pressure from 250 mbar and 20~30°C to 30 mbar and 30°C.

(5. Purification step-filtration) After that, the residue from which diethyl carbonate (DEC) was removed was washed using methylene chloride(MC) of twice the weight of PC used at 20~30°C, and vacuum-filtered.

(6-1. Additional purification step-redissolving step) After that, bisphenol A was added to 16.6 mol of ethanol, and re-dissolved.

(6-2. Additional purification step-adsorption step) After that, lignite activated carbon as an adsorbent was added at a ratio of 30 wt.% relative to waste polycarbonate, purified through an adsorption tower for 3 hours, and then filtered to remove the lignite activated carbon.

(6-3. Additional purification step - recrystallization step) After that, water was added and bisphenol A was recrystallized, and the resulting slurry was vacuum-filtered at 20~30°C to recover bisphenol A (BPA) crystals.

(7. Drying step) After that, the result was vacuum-dried in a convection oven at 40°C to prepare a recycled bisphenol A monomer composition in which recycled bisphenol A (BPA) has been recovered.

Example 2

A recycled bisphenol A monomer composition was prepared in the same manner as in Example 1, except that (1. Decomposition step) of Example 1 was changed as follows.

(1. Decomposition step) 1 mol of waste polycarbonate(PC) was added to a reactor 1 and dissolved using methylene chloride(MC), and then and foreign materials were filtered out. Then, 11 mol of ethanol (EtOH), 0.2 mol of sodium hydroxide(NaOH), and an antioxidant were added thereto and stirred at 80°C to perform a PC depolymerization reaction.

During the PC depolymerization reaction, an in-line analyzer 2, i.e., a Raman probe, was inserted into the reactor 1, and set so that a detection unit 3 was not disturbed due to the viscosity of the polycarbonate(PC) during stirring. The Raman spectrum was measured at regular intervals (about 3 minutes) for a total of 6 hours after the start of the reaction, and analyzed in real time using a computer 4, and the reaction was terminated at the optimal reaction point (BPA/MC Raman integral ratio = 0.12).

**<Experimental Example>**

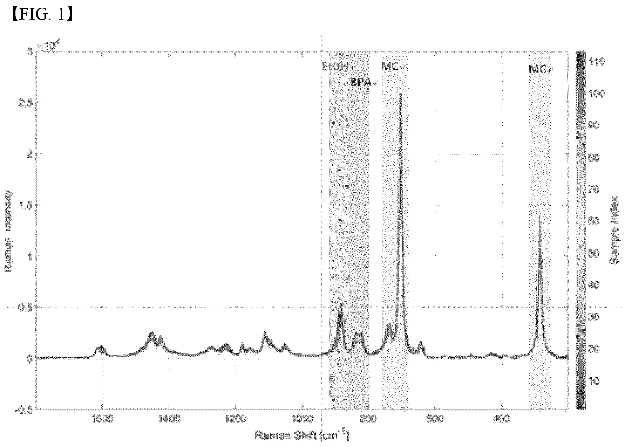

The physical properties were measured by the following methods, and the results are shown in Tables 1 and 2, and FIGS. 1 and 2.

**1. Raman spectrum analysis results**

(1) Integral ratio for aromatic diol compound

In the (1. Decomposition step) of Examples, the integral ratio for bisphenol A (BPA) as the aromatic diol compound was obtained through an in-line Raman analyzer at regular time intervals (about 3 minutes) for a total of 6 hours after the start of the PC depolymerization reaction. The integral ratios were shown in Table 1 and FIG. 2 for Example 1, and in Table 2 for Example 2. Specifically, the integral ratio for the aromatic diol compound(BPA) can be calculated by dividing the integral value of BPA band (791.2 $cm^{-1}$ to 861.5 $cm^{-1}$) by the integral value of MC band (663.4 $cm^{-1}$ to 785.1 cm-1 and 234.8 cm-1 to 337.1 cm-1) according to the following Mathematical Formula 1:

Integral ratio for aromatic diol compound = {Integral value of the region from 791.2 $cm^{-1}$ to 861.5 $cm^{-1}$ on the Raman spectrum / (Integral value of the region from 663.4 $cm^{-1}$ to 785.1 $cm^{-1}$ on the Raman spectrum + Integral value of the region from 234.8 $cm^{-1}$ to 337.1 $cm^{-1}$ on the Raman spectrum)}     [Mathematical Formula 1]

in the Mathematical Formula 1, the region from 791.2 cm$^{-1}$ to 861.5 cm$^{-1}$ on the Raman spectrum is the spectrum of bisphenol A(BPA), and the region from 663.4 cm$^{-1}$ to 785.1 cm$^{-1}$ and from 234.8 cm$^{-1}$ to 337.1 cm$^{-1}$ on the Raman spectrum is the spectrum of methylene chloride(MC).

The Raman analysis was performed using MarqMetrix All-in-one apparatus and BallProbe, and the spectrum inside the reactor was measured in real time under Excitation Laser (785 nm, 400 Mw) conditions, and the baseline correction progressed linearly in the regions of each component of MC and BPA.

(2) Integral ratio for alcohol

In the (1. Decomposition step) of Examples, the integral ratio for ethanol (EtOH) as an alcohol was obtained through an in-line Raman analyzer at regular time intervals (about 3 minutes) for a total of 6 hours after the start of the PC depolymerization reaction. The integral ratios were shown in Table 1 and FIG. 2 for Example 1, and in Table 2 for Example 2. Specifically, the integral ratio for the alcohol (EtOH) can be calculated by dividing the integral value of EtOH band (849.7 cm$^{-1}$ to 910 cm$^{-1}$) by the integral value of MC band (663.4 cm$^{-1}$ to 785.1 cm$^{-1}$, and 234.8 cm$^{-1}$ to 337.1 cm$^{-1}$) according to the following Mathematical Formula 2:

Integral ratio for alcohol = {Integral value of the region from 849.7 cm$^{-1}$ to 910 cm$^{-1}$ on the Raman spectrum / (Integral value of the region from 663.4 cm$^{-1}$ to 785.1 cm$^{-1}$ on the Raman spectrum + Integral value of the region from 234.8 cm$^{-1}$ to 337.1 cm$^{-1}$ on the Raman spectrum)}    [Mathematical Formula 2]

in the Mathematical Formula 2, the region from 849.7 cm$^{-1}$ to 910 cm$^{-1}$ on the Raman spectrum is the spectrum of ethanol(EtOH), and the region from 663.4 cm$^{-1}$ to 785.1 cm$^{-1}$ and from 234.8 cm$^{-1}$ to 337.1 cm$^{-1}$ on the Raman spectrum are the spectra of methylene chloride(MC).

The Raman analysis was performed using MarqMetrix All-in-one apparatus and BallProbe, and the spectrum inside the reactor was measured in real time under Excitation Laser (785 nm, 400 Mw) conditions, and the baseline correction progressed linearly in the regions of each component of EtOH and BPA.

**2. BPA Purity**

In the (1. Decomposition step) of Examples, the depolymerization reaction was performed at regular time intervals (about 3 minutes) for a total of 6 hours after the start of the PC depolymerization reaction, and the weight of the bisphenol A (BPA) produced from the reaction was confirmed through HPLC measurement. The weight of BPA generated when the polycarbonate used in the reaction was 100% decomposed was measured, and the yield of BPA was calculated according to the following Equation 4.

[Equation 4]

$$\text{Yield (\%)} = (W_1/W_0) * 100$$

In Equation 4, $W_0$ is the mass of BPA obtained at 100% decomposition, and $W_1$ is the mass of BPA actually obtained. Specifically, when about 100 g of polycarbonate is decomposed, the mass of BPA theoretically obtained at 100% decomposition is 89 g. If the mass of BPA actually obtained is 80 g, the yield is (80/89) * 100 = 90%.

[Table 1]

| Experimental Example measurement results for Example 1 | | | |
|---|---|---|---|
| Measurement result (min) | Mathematical Formula 1 | Mathematical Formula 2 | BPA yield (%) |
| 3 | 0.05 | 0.18 | 9.8 |
| 6 | 0.082 | 0.179 | 16.8 |
| 16 | 0.101 | 0.17 | 30.9 |
| 25 | 0.105 | 0.11 | 55.8 |
| 36 | 0.106 | 0.105 | 66.2 |
| 55 | 0.111 | 0.10 | 78.3 |
| 70 | 0.112 | 0.098 | 82.1 |

(continued)

| Experimental Example measurement results for Example 1 | | | |
|---|---|---|---|
| Measurement result (min) | Mathematical Formula 1 | Mathematical Formula 2 | BPA yield (%) |
| 80 | 0.113 | 0.097 | 83.6 |
| 120 | 0.12 | 0.095 | 90.1 |

[Table 2]

| Experimental Example measurement results for Example 2 | | |
|---|---|---|
| Measurement result (min) | Mathematical Formula 1 | BPA yield (%) |
| 3 | 0.06 | 9.3 |
| 6 | 0.09 | 17.1 |
| 16 | 0.105 | 31.1 |
| 25 | 0.11 | 65.4 |
| 60 | 0.115 | 88.1 |
| 70 | 0.116 | 89.9 |
| 80 | 0.118 | 90.1 |
| 90 | 0.12 | 90.2 |
| 100 | 0.122 | 92.1 |

**Claims**

1. A preparation method of a monomer composition for synthesizing recycled plastic, the method comprising the steps of:

   subjecting a polycarbonate-based resin to a depolymerization reaction in the presence of an alcohol;
   monitoring a Raman spectrum of an aromatic diol compound obtained from the depolymerization reaction or the alcohol in a reactor through which the depolymerization reaction proceeds; and
   recovering the aromatic diol compound.

2. The preparation method of a monomer composition for synthesizing recycled plastic according to claim 1, wherein:

   the monitoring a Raman spectrum of an aromatic diol compound obtained from the depolymerization reaction or the alcohol in a reactor through which the depolymerization reaction proceeds,
   is performed through a Raman spectrum measuring apparatus inserted into the reactor.

3. The preparation method of a monomer composition for synthesizing recycled plastic according to claim 1, wherein:

   the monitoring a Raman spectrum of an aromatic diol compound obtained from the depolymerization reaction or the alcohol in a reactor through which the depolymerization reaction proceeds,
   comprises measuring an Integral ratio for the aromatic diol compound according to the following Mathematical Formula 1:

   Integral ratio for aromatic diol compound = {Integral value of the region from --> $791.2\ cm^{-1}$ to $861.5\ cm^{-1}$ on the Raman spectrum / (Integral value of the region from $663.4\ cm^{-1}$ to $785.1\ cm^{-1}$ on the Raman spectrum + Integral value of the region from $234.8\ cm^{-1}$ to $337.1\ cm^{-1}$ on the Raman spectrum)}        [Mathematical Formula 1]

   in the Mathematical Formula 1, the region from $791.2\ cm^{-1}$ to $861.5\ cm^{-1}$ on the Raman spectrum is the spectrum of the aromatic diol compound, and

the region from 663.4 cm$^{-1}$ to 785.1 cm$^{-1}$ and from 234.8 cm$^{-1}$ to 337.1 cm$^{-1}$ on the Raman spectrum is the spectrum of a reaction solvent.

4. The preparation method of a monomer composition for synthesizing recycled plastic according to claim 3, wherein: when the integral ratio for an aromatic diol compound according to the Mathematical Formula 1 is 0.115 to 0.125, the depolymerization reaction is terminated.

5. The preparation method of a monomer composition for synthesizing recycled plastic according to claim 1, wherein:

the monitoring a Raman spectrum of an aromatic diol compound obtained from the depolymerization reaction or the alcohol in a reactor through which the depolymerization reaction proceeds,
comprises measuring an integral ratio for an alcohol according to the following Mathematical Formula 2:

Integral ratio for alcohol = {Integral value of the region from 849.7 cm$^{-1}$ to 910 cm$^{-1}$ on the Raman spectrum / (Integral value of the region from 663.4 cm$^{-1}$ to 785.1 cm$^{-1}$ on the Raman spectrum + Integral value of the region from 234.8 cm$^{-1}$ to 337.1 cm$^{-1}$ on the Raman spectrum)}     [Mathematical Formula 2]

in the Mathematical Formula 2, the region from 849.7 cm$^{-1}$ to 910 cm$^{-1}$ on the Raman spectrum is the spectrum of the alcohol, and
the region from 663.4 cm$^{-1}$ to 785.1 cm$^{-1}$ and from 234.8 cm$^{-1}$ to 337.1 cm$^{-1}$ on the Raman spectrum is the spectrum of a reaction solvent.

6. The preparation method of a monomer composition for synthesizing recycled plastic according to claim 5, wherein: when the integral ratio for an alcohol according to the Mathematical Formula 2 is 0.09 to 0.1, the depolymerization reaction is terminated.

7. The preparation method of a monomer composition for synthesizing recycled plastic according to claim 1, wherein: the aromatic diol compound is bisphenol A.

8. The preparation method of a monomer composition for synthesizing recycled plastic according to claim 1, wherein: the alcohol is ethanol.

9. The preparation method of a monomer composition for synthesizing recycled plastic according to claim 1, wherein:

the monitoring a Raman spectrum of an aromatic diol compound obtained from the depolymerization reaction or the alcohol in a reactor through which the depolymerization reaction proceeds,
further comprises monitoring the Raman spectrum of impurities derived from the aromatic diol compound produced by the depolymerization reaction.

10. The preparation method of a monomer composition for synthesizing recycled plastic according to claim 9, wherein:

the monitoring the Raman spectrum of impurities derived from an aromatic diol compound produced by the depolymerization reaction,
comprises measuring the integral ratio for impurities derived from an aromatic diol compound according to the following Mathematical Formula 3:

Integral ratio for impurities derived from aromatic diol compound = {Integral value of the region from 800 cm$^{-1}$ to 850 cm$^{-1}$ on the Raman spectrum / (Integral value of the region from 663.4 cm$^{-1}$ to 785.1 cm$^{-1}$ on the Raman spectrum + Integral value of the region from 234.8 cm$^{-1}$ to 337.1 cm$^{-1}$ on the Raman spectrum)}     [Mathematical Formula 3]

in the Mathematical Formula 3, the region from 800 cm$^{-1}$ to 850 cm$^{-1}$ on the Raman spectrum is the spectrum of impurities derived from aromatic diol compound, and
the region from 663.4 cm$^{-1}$ to 785.1 cm$^{-1}$ and from 234.8 cm$^{-1}$ to 337.1 cm$^{-1}$ on the Raman spectrum is the spectrum of a reaction solvent.

11. The preparation method of a monomer composition for synthesizing recycled plastic according to claim 10, wherein: when the integral ratio for impurities derived from an aromatic diol compound according to the Mathematical Formula 3 is greater than 0.12, the depolymerization reaction is terminated.

12. The preparation method of a monomer composition for synthesizing recycled plastic according to claim 1, wherein:

the monitoring a Raman spectrum of an aromatic diol compound obtained from the depolymerization reaction or the alcohol in a reactor through which the depolymerization reaction proceeds, is performed repeatedly at intervals of 5 minutes or less.

13. A preparation device of a monomer composition for synthesizing recycled plastic, comprising:

a reactor that subjects a polycarbonate-based resin to a depolymerization reaction in the presence of an alcohol; an in-line analyzer that is inserted in the reactor to monitor a Raman spectrum of an aromatic diol compound obtained from the depolymerization reaction or alcohol; and a recovery unit that recovers the aromatic diol compound obtained from the depolymerization reaction.

14. The preparation device of a monomer composition for synthesizing recycled plastic according to claim 13, wherein: the in-line analyzer comprises a Raman spectrum measuring apparatus.

15. The preparation device of a monomer composition for synthesizing recycled plastic according to claim 13, further comprising a computer that analyzes the Raman spectrum measured by the in-line analyzer.

16. The preparation device of a monomer composition for synthesizing recycled plastic according to claim 13, wherein: the in-line analyzer comprises a detection unit that comes into contact with reactants in the reactor.

17. A monomer composition for synthesizing recycled plastic, comprising an aromatic diol compound obtained by the preparation method of a monomer composition for synthesizing recycled plastic according to claim 1, or the preparation device of a monomer composition for synthesizing recycled plastic according to claim 13.

18. A recycled plastic comprising a reaction product of the monomer composition for synthesizing recycled plastic according to claim 17 and a comonomer.

19. A molded product comprising the recycled plastic according to claim 18.

【FIG. 1】

【FIG. 2】

【FIG. 3】

## INTERNATIONAL SEARCH REPORT

| International application No. |
|---|
| **PCT/KR2024/010372** |

| A. | CLASSIFICATION OF SUBJECT MATTER |
|---|---|

**C08J 11/24**(2006.01)i; **C07C 68/06**(2006.01)i; **C07C 67/54**(2006.01)i; **C07C 7/12**(2006.01)i; **C07C 7/04**(2006.01)i

According to International Patent Classification (IPC) or to both national classification and IPC

| B. | FIELDS SEARCHED |
|---|---|

Minimum documentation searched (classification system followed by classification symbols)

C08J 11/24(2006.01); B01F 27/91(2022.01); B01F 35/00(2022.01); C07C 37/055(2006.01); C07C 37/68(2006.01); C07C 68/06(2006.01); C08G 64/20(2006.01); C08G 64/24(2006.01); G01J 3/44(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폴리카보네이트(polycarbonate), 해중합(depolymerization), 비스페놀 A(bisphenol A), 라만(raman)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2011-0134488 A (STYRON EUROPE GMBH) 14 December 2011 (2011-12-14) See claims 1-10; paragraphs [0004]-[0043]; and figure 3. | 1-19 |
| A | KR 10-2015-0096791 A (SAUDI BASIC INDUSTRIES CORPORATION) 25 August 2015 (2015-08-25) See claims 1-23. | 1-19 |
| A | US 8432544 B2 (YAO, W. et al.) 30 April 2013 (2013-04-30) See claims 1-4. | 1-19 |
| A | US 6608678 B1 (POTYRAILO, R. A. et al.) 19 August 2003 (2003-08-19) See claims 1-70. | 1-19 |
| A | KR 10-2023-0039955 A (LG CHEM, LTD.) 22 March 2023 (2023-03-22) See claims 1-15. | 1-19 |

☐ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **28 October 2024** | **28 October 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2024/010372**

| Patent document cited in search report | | | Publication date (day/month/year) | Patent family member(s) | | | Publication date (day/month/year) |
|---|---|---|---|---|---|---|---|
| KR | 10-2011-0134488 | A | 14 December 2011 | CN | 102361910 | A | 22 February 2012 |
| | | | | CN | 102361910 | B | 16 October 2013 |
| | | | | CN | 102847164 | A | 02 January 2013 |
| | | | | CN | 202891969 | U | 24 April 2013 |
| | | | | EP | 2411447 | A1 | 01 February 2012 |
| | | | | EP | 2411447 | B1 | 03 July 2013 |
| | | | | JP | 2012-521482 | A | 13 September 2012 |
| | | | | JP | 5647219 | B2 | 24 December 2014 |
| | | | | SG | 174169 | A1 | 28 November 2011 |
| | | | | US | 2012-0041158 | A1 | 16 February 2012 |
| | | | | US | 8426531 | B2 | 23 April 2013 |
| | | | | WO | 2010-110984 | A1 | 30 September 2010 |
| KR | 10-2015-0096791 | A | 25 August 2015 | CN | 104870418 | A | 26 August 2015 |
| | | | | CN | 104870418 | B | 14 December 2016 |
| | | | | EP | 2746249 | A1 | 25 June 2014 |
| | | | | EP | 2746249 | B1 | 07 June 2017 |
| | | | | JP | 2016-505591 | A | 25 February 2016 |
| | | | | US | 2015-0291763 | A1 | 15 October 2015 |
| | | | | US | 9428627 | B2 | 30 August 2016 |
| | | | | WO | 2014-099550 | A1 | 26 June 2014 |
| US | 8432544 | B2 | 30 April 2013 | CN | 101995400 | A | 30 March 2011 |
| | | | | CN | 101995400 | B | 11 January 2012 |
| | | | | US | 2012-0236300 | A1 | 20 September 2012 |
| | | | | WO | 2012-040990 | A1 | 05 April 2012 |
| | | | | WO | 2012-040990 | A8 | 29 November 2012 |
| US | 6608678 | B1 | 19 August 2003 | AU | 2002-861101 | A | 29 April 2002 |
| | | | | WO | 02-33388 | A1 | 25 April 2002 |
| KR | 10-2023-0039955 | A | 22 March 2023 | | None | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230109847